# EUROPÄISCHE PATENTSCHRIFT

(11) **EP 3 099 656 B1**
(45) Veröffentlichungstag und Bekanntmachung des Hinweises auf die Patenterteilung: **14.03.2018**
(21) Anmeldenummer: 15707553.2
(22) Anmeldetag: 26.01.2015
(51) Int. Cl.: C07C 29/141, C07C 45/75

(54) **VERFAHREN ZUR HERSTELLUNG VON NEOPENTYLGLYKOL**
PROCESS FOR PRODUCING NEOPENTYL GLYCOL
PROCÉDÉ DE PRODUCTION DE NÉOPENTYLGLYCOL

(30) Priorität: 28.01.2014 DE 102014100996
(43) Veröffentlichungstag der Anmeldung: 07.12.2016
(73) Patentinhaber: OXEA GmbH, 46147 Oberhausen (DE)
(72) Erfinder: EISENACHER, Matthias, 46569 Hünxe (DE); SCHALAPSKI, Kurt, 46147 Oberhausen (DE); STRUTZ, Heinz, 47445 Moers (DE)
(74) Vertreter: Michalski Hüttermann & Partner Patentanwälte mbB
(86) Internationale Anmeldenummer: PCT/EP2015/051466
(87) Internationale Veröffentlichungsnummer: WO 2015/113928

(56) Entgegenhaltungen:
- WO-A1-2008/107333
- WO-A2-2010/000382
- DE-A1- 10 317 545

## Beschreibung

Die vorliegende Erfindung bezieht sich auf ein Verfahren zur Herstellung von Neopentylglykol.

Neopentylglykol (=2,2-Dimethyl-1,3-propandiol) ist eine wichtige Verbindung der industriellen Chemie, hauptsächlich als Rohstoff für die Herstellung gesättigter Polyesterharze für Pulverlacke sowie für glasfaserverstärkte Kunststoffe.

Ein mögliches Verfahren zur Herstellung von Neopentylglykol beschreibt beispielsweise die DE10317545 A1. Diese offenbart ein Verfahren zur gleichzeitigen Herstellung von Hydroxypivalinsäureneopentylglykolester (HPN) und Neopentylglykol, wobei a) Isobutyraldehyd und Formaldehyd in Gegenwart von tertiären Aminen zu einer Hydroxypivalinaldehyd enthaltenden Lösung umgesetzt werden, b) Leichtsieder aus dieser Lösung durch Destillation abgetrennt und in die Aldolisierung a) zurückgeführt werden, c) das so erhaltene Sumpfprodukt in zwei Teilströme aufgeteilt wird, d) von denen der eine Teilstrom zu Neopentylglykol hydriert e) und der andere Teilstrom zu HPN umgesetzt wird.

Des Weiteren offenbart die WO 2008/107333 A1 ein Verfahren zur Herstellung von Hydroxypivalinaldehyd durch Aldolisierung von Isobutyraldehyd mit Formaldehyd sowie ein Verfahren zur Herstellung von Neopentylglykol durch Hydrierung des so erhaltenen lsobutyraldehyds.

Trotzdem wird ständig nach neuen, verbesserten Herstellungsverfahren für Neopentylglykol gesucht.

Es ist somit eine Aufgabe, ein neues Herstellungsverfahren von Neopentylglykol bereitzustellen. Diese Aufgabe wird durch ein Verfahren gemäß Anspruch 1 der vorliegenden Erfindung gelöst. Demgemäß wird ein Verfahren bereitgestellt, umfassend die Schritte:
a) Umsetzung von Isobutyraldehyd mit Formaldehyd in Gegenwart eines basischen Katalysators, wobei der Isobutyraldehyd im Überschuss vorliegt
b) Destillative Aufreinigung des Reaktionsgemisches aus a), so dass ein Sumpfprodukt entsteht, welches ≤5% Gew.% Wasser enthält
c) Hydrierung des Sumpfprodukts zu Neopentylglykol

Überraschenderweise wurde herausgefunden, dass durch eine Umsetzung im Basischen, wobei der Isobutyraldehyd im Überschuss vorliegt, bei der anschließenden destillativen Aufreinigung ein Sumpfprodukt erhalten werden kann, dass 5% und weniger Wasser enthält und bei den meisten Anwendungen obendrein vollständig vom Katalysator der vorrausgegangenen Aldolreaktion befreit ist. Ein derartiges Sumpfprodukt kann anschließend so hydriert werden, dass nicht nur Hydroxypivalinaldehyd selektiv zu Neopentylglycol umgesetzt wird, sondern auch hochsiedende Verunreinigungen sehr selektiv zu Neopentylglycol umgesetzt werden können.

Die einzelnen Schritte werden im Folgenden noch näher erläutert:

### Schritt a) Umsetzung von Isobutyraldehyd mit Formaldehyd

Diese Umsetzung erfolgt bevorzugt bei einer Temperatur von ≥40 °C bis ≤100°C und kann kontinuerlich oder diskontinuerlich erfolgen.

Bei den allermeisten Anwendungen wird Formaldehyd in Form wäßriger Formaldehydlösung verwendet.

Der Isobutyraldehyd liegt dabei gegenüber dem Formaldehyd im Überschuss vor, bevorzugt ist ein Verhältnis von ≥1.01:1 (mol Isobutyraldehyd zu mol Formaldehyd), noch bevorzugt ≥1.03:1, ferner bevorzugt ≥1.05:1 bis ≤1.2:1, am meisten bevorzugt ≥1.1:1 bis ≤1.15:1.

Schritt a) erfolgt in Gegenwart eines basischen Katalysators, wobei der basische Katalysator bevorzugt im molaren Verhältnis von ≥0.01 bis ≤0.1, bezogen auf den Isobutyraldehyd eingesetzt wird.

Bevorzugt umfasst der basische Katalysator dabei Trimethylamin und/oder wäßrige Alkalilösung, bevorzugt Natron- und/oder Kalilauge.

Umfasst der basische Katalysator wäßrige Alkalilösung, so erfolgt bevorzugt nach Schritt a) ein Schritt a1):
a1) Abtrennung der wässrige Phase vor der Durchführung von Schritt b)

### Schritt b) Destillative Aufreinigung

Schritt b) erfolgt bevorzugt im Dünnschichtverdampfer. Dieser verfügt bevorzugt über eine aufgesetzte Kolonne mit 10 bis 30 Böden. Weiterhin bevorzugt sind Temperaturen von ≥170°C bis ≤200°C.

Da in Schritt a) der Isobutyraldehyd im Überschuss vorliegt, entsteht bei der Destillation im Destillat ein Zweiphasensystem, bestehend aus einer organischen Phase, welche i.w. Isobutyraldehyd enthält und einer wäßrigen Phase; dies erlaubt, den Isobutyraldehyd ohne weitere Aufreinigungsschritte wieder Schritt a) zurückzuführen.

Gemäß einer bevorzugten Ausführungsform der Erfindung umfasst das Verfahren somit einen Schritt b1), der nach Schritt b) durchgeführt wird:
b1) Rückführen der in Schritt b) abgetrennten organischen, Isobutyraldehyd enthaltenen Phase zur erneuten Reaktion gemäß Schritt a)

Schritt b) wird bevorzugt so durchgeführt, dass das Sumpfprodukt ≤3, noch bevorzugt ≤2 Gew.-% Wasser enthält.

Gemäß einer bevorzugten Ausführungsform der vorliegenden Erfindung wird die organische Phase mindestens auf dem 6. Boden der aufgesetzten Kolonne zugeführt. Es hat sich herausgestellt, dass so eine besonders gute Trennleistung möglich ist.

### Schritt c) Hydrierung

Bevorzugt erfolgt Schritt c) direkt nach Schritt b), d.h. gemäß einer bevorzugten Ausführungsform der Erfindung findet zwischen Schritt b) und c) keine weitere Aufreinigung des Destillationssumpfes statt.

Bevorzugt findet Schritt c) bei einem Druck von ≥6 MPa bis ≤20 MPa Wasserstoff statt, noch bevorzugt ≥8 MPa bis ≤18 MPa.

Bevorzugt findet Schritt c) bei einer Temperatur von ≥100°C bis ≤220°C statt, dies hat sich in der Praxis bewährt.

Schritt c) kann in einem einstufigen Reaktor durchgeführt werden. Besonders bevorzugt ist jedoch eine zwei- oder mehrstufige Hydrierung in einem Mehrzonenreaktor. Hierbei sind die Druckverhältnisse (s.o.) dann jeweils die Druckverhältnisse über den gesamten Reaktor.

Ist eine zweistufige Hydrierung gewählt, so findet diese bevorzugt so statt, dass in der Zone des Reaktors, welche zuerst vom Hydriergut erreicht wird bei ≥100 bis ≤140 °C und einer V/Vh von ≥0,7 - ≤1,0 h⁻¹ (bezogen nur auf die erste Zone des Reaktors) gearbeitet wird und in der darauffolgenden Zone des Reaktors bei ≥150 bis ≤220 °C und einer V/Vh von ≥0,2 - ≥0,8 h⁻¹ (bezogen nur auf die zweite Zone des Reaktors) gearbeitet wird. Dies hat sich in der Praxis bewährt.

Der Katalysator umfasst bevorzugt einen Katalysator auf Nickel und/oder Kupferchromitbasis, bevorzugt mit Mangan und/oder Bariumdotierung. Dies hat sich in der Praxis bewährt.

Das Produkt kann anschließend je nach Anwendung und konkretem Einsatzgebiet des Verfahrens noch weiteren Aufreinigungsschritten wie etwa spezifikationsgerechtes Aufdestillieren etc. unterworfen werden. Dies stellen ebenfalls bevorzugte Ausführungsformen der vorliegenden Erfindung dar.

Die vorgenannten sowie die beanspruchten und in den Ausführungsbeispielen beschriebenen erfindungsgemäß zu verwendenden Bauteile unterliegen in ihrer Größe, Formgestaltung, Trägermaterialauswahl und technischen Konzeption keinen besonderen Ausnahmebedingungen, so dass die in dem Anwendungsgebiet bekannten Auswahlkriterien uneingeschränkt Anwendung finden können.

Weitere Einzelheiten, Merkmale und Vorteile des Gegenstandes der Erfindung ergeben sich aus den Unteransprüchen sowie aus der nachfolgenden Beschreibung des folgenden Beispiels, welches rein illustrativ zu verstehen ist.

### BEISPIEL

### Herstellung des verwendeten Hydrierkatalysators

2,8 kg Kupfernitrat-Trihydrat, 400 g Mangannitrat (50%ige Lösung in verdünnter Salpetersäure) und 150 g Bariumnitrat werden in 20 L dest. H₂O bei 55 °C gelöst. Separat werden 2,6 kg Ammoniumdichromat in 12 L Wasser und 4 L 25%iger Ammoniaklösung gelöst.

Danach wird die Ammoniumdichromat-Lösung langsam in die Kupfernitratlösung getropft. Dabei fällt ein rot-brauner Feststoff aus. Zur Vervollständigung der Fällung wird noch eine Stunde nachgerührt und dabei auf Raumtemperatur abgekühlt. Anschließend wird der Feststoff abfiltriert. Daraufhin wird der Feststoff bei 110 °C im Trockenschrank getrocknet. Der trockene Feststoff wird bei 350 °C vier Stunden kalziniert, die Aufheizrate beträgt dabei 2°C/min.

Nach dem Kalzinieren und wieder Erkalten des Feststoffs wird dieser mit 20 L 10%iger Essigsäure aufgerührt. Danach wird der Feststoff mit Wasser säurefrei gewaschen und erneut bei 110 °C getrocknet und bei 350 °C (Aufheizrate 2°C/min) geglüht.

Anschließend ist der Feststoff als Katalysator einsetzbar.

Auf die Metalle bezogen wies der Katalysator folgende Zusammensetzung, bezogen auf den Anteil Kupfer, Chrom, Mangan und Barium, auf:
47,5% Kupfer, 46,5% Chrom, 4,0% Mangan, 2,0% Barium

### Durchführung des erfindungsgemäßen Verfahrens:

3996 g Isobutyraldehyd (97,4%-ig) und 3034 g Formalin (49%-ige wässrige Lösung) werden in einen Autoklaven gegeben und auf 45 °C erwärmt. Anschließend werden 151 g Trimethylamin (40%-ige wässrige Lösung) zugepumpt. Sobald die Zugabe beendet ist, wird das Reaktionsgemisch auf 90 °C erwärmt und eine Stunde bei dieser Temperatur belassen. Anschließend wird das Produkt abgelassen. Die Zusammensetzung des Produktes lautet:

| *Substanz* | *Anteil* / *g* |
|---|---|
| Trimethylamin | 60 |
| Isobutyraldehyd | 225 |
| Methanol | 10 |
| Hydroxypivalinaldehyd | 4931 |
| Neopentylglycol-monoisobutyrat | 26 |
| Neopentylglycol | 29 |
| Hydroxypivalinsäureneopentylglyco lester | 151 |
| Wasser | 1720 |
| Sonstige organische Verbindungen | 29 |

Dieses Gemisch wird anschließend in einem Dünnschichtverdampfer mit aufgesetzter Kolonne destillativ aufbereitet. Dabei wird das Gemisch auf Boden 20 einer 26 Böden-Füllkörperkolonne zugeführt. Der Dünnschichtverdampfer wird bei 170 °C betrieben. Bei dieser Aufarbeitung fallen ein zwei-phasiger Destillationskopf und ein Destillationssumpf an. Die organische Phase des Destillationskopfes weist folgende Zusammensetzung auf:

| *Substanz* | *Anteil* / *g* |
|---|---|
| Trimethylamin | 23 |
| Isobutyraldehyd | 185 |
| Hydroxypivalinaldehyd | 102 |
| Wasser | 11 |
| Sonstige organische Verbindungen | 37 |

Diese organische Phase kann anschließend ohne weitere Aufarbeitung erneut in die Aldolisierung eingesetzt werden.

Die wässrige Phase des Destillationskopfes weist folgende Zusammensetzung auf:

| *Substanz* | *Anteil* / *g* |
|---|---|
| Trimethylamin | 37 |
| Isobutyraldehyd | 25 |
| Hydroxypivalinaldehyd | 8 |
| Wasser | 1598 |
| Sonstige organische Verbindungen | 23 |

Diese wässrige Phase wird anschließend verworfen.

Der Destillationssumpf weist folgende Zusammensetzung auf:

| *Substanz* | *Anteil* / *g* |
|---|---|
| Isobutyraldehyd | 15 |
| Hydroxypivalinaldehyd | 4776 |
| Cyclisches Acetal aus Neopentylglycol und Hydroxypivalinaldehyd | 16 |
| Neopentylglycol-monoisobutyrat | 25 |
| Neopentylglycol | 23 |
| Hydroxypivalinsäureneopentylglycolester | 212 |
| Wasser | 111 |
| Sonstige organische Verbindungen | 88 |

Der Destillationssumpf wird anschließend hydriert, dies geschieht folgendermaßen:
Der oben angegebene Katalysator wird mit 3% Graphit vermischt und tablettiert. Die erhaltenen 5x5 mm Tabletten werden in einen Rohrreaktor mit 1,3 Liter Volumen gegeben. Dabei ist der Reaktor so ausgestattet, dass die unteren 0,3 Liter der Katalysatorschüttung separat beheizt werden können und der obere 1,0 Liter der Katalysatorschüttung ebenfalls separat beheizt werden kann.

Zur Katalysatoraktivierung werden beiden Katalysatorbetten jeweils auf die gleiche Temperatur geheizt. Der Katalysator wird wie folgt aktiviert:
Aufheizrate 20°C/h bis 180 °C,

| | |
|---|---|
| Stickstoff | 1000 NL/h |
| Wasserstoff | 20 NL/h |
| Dauer 12 h | |
| | |
| Stickstoff | 1000 NL/h |
| Wasserstoff | 60 NL/h |
| Dauer 6 h | |
| | |
| Stickstoff | 1000 NL/h |
| Wasserstoff | 120 NL/h |
| Dauer 6 h | |

Anschließend wird die untere Schüttung auf 130 °C und die obere Schüttung auf 170 °C aufgeheizt, ein Wasserstoffdruck von 8 MPa angelegt und 300 mL/h des Destillationssumpfes aus Beispiel 2 von unten in den Reaktor gefördert. Das daraus erhaltene Produkt weist die folgende Zusammensetzung auf:

| *Substanz* | *Anteil* / *g* |
|---|---|
| Isobutanol | 25 |
| Neopentylglycol | 5000 |
| Hydroxypivalinsäureneopentylglycolester | 21 |
| Wasser | 111 |
| Sonstige organische Verbindungen | 108 |

Das Produkt kann anschließend nach den bekannten Verfahren aufgereinigt werden.

## Patentansprüche

1. Verfahren zur Synthese von Neopentylglykol, umfassend die Schritte:
a) Umsetzung von Isobutyraldehyd mit Formaldehyd in Gegenwart eines basischen Katalysators, wobei der Isobutyraldehyd im Überschuss vorliegt
b) Destillative Aufreinigung des Reaktionsgemisches aus a), so dass ein Sumpfprodukt entsteht, welches weniger als 5% Gew.% Wasser enthält
c) Hydrierung des Sumpfprodukts zu Neopentylglykol

2. Verfahren nach Anspruch 1, wobei das Verhältnis von Isobutyraldehyd zu Formaldehyd ≥1.01:1 (mol Isobutyraldehyd zu mol Formaldehyd) beträgt.

3. Verfahren nach Anspruch 1 oder 2, wobei der basische Katalysator im molaren Verhältnis von ≥0.01 bis ≤0.1, bezogen auf den Isobutyraldehyd eingesetzt wird.

4. Verfahren nach einem der Ansprüche 1 bis 3, wobei der basische Katalysator Trimethylamin und/oder Alkalilauge umfasst.

5. Verfahren nach einem der Ansprüche 1 bis 4, wobei Schritt b) im Dünnschichtverdampfer erfolgt.

6. Verfahren nach einem der Ansprüche 1 bis 5, zusätzlich umfassend den Schritt b1), der nach Schritt b) durchgeführt wird:
b1) Rückführen der in Schritt b) abgetrennten organischen, Isobutyraldehyd enthaltenen Phase zur erneuten Reaktion gemäß Schritt a)

7. Verfahren nach einem der Ansprüche 1 bis 6, wobei zwischen Schritt b) und c) keine weitere Aufreinigung des Destillationssumpfes stattfindet.

8. Verfahren nach einem der Ansprüche 1 bis 7, wobei Schritt c) bei einem Druck von ≥6 MPa bis ≤20 MPa Wasserstoff stattfindet.

9. Verfahren nach einem der Ansprüche 1 bis 8, wobei Schritt c) als zwei- oder mehrstufige Hydrierung in einem Mehrzonenreaktor durchgeführt wird.

10. Verfahren nach einem der Ansprüche 1 bis 9, wobei Schritt c) einen Katalysator auf Nickel und/oder Kupferchromitbasis umfasst.

## Claims

1. Process for the synthesis of neopentyl glycol comprising the steps of:
a) reacting isobutyraldehyde with formaldehyde in the presence of a basic catalyst, wherein the isobutyraldehyde is present in excess
b) purifying the reaction mixture from a) by distillation resulting in a bottom product comprising less than 5% by weight water
c) hydrogenating the bottom product to give neopentyl glycol

2. Process according to Claim 1, wherein the ratio of isobutyraldehyde to formaldehyde is ≥ 1.01:1 (mol of isobutyraldehyde to mol of formaldehyde).

3. Process according to Claim 1 or 2, wherein the basic catalyst is used in a molar ratio of ≥ 0.01 to ≤ 0.1, based on isobutyraldehyde.

4. Process according to any of Claims 1 to 3, wherein the basic catalyst comprises trimethylamine and/or alkali metal hydroxide solution.

5. Process according to any of Claims 1 to 4, wherein step b) is conducted in a thin film evaporator.

6. Process according to any of Claims 1 to 5 additionally comprising step b1), which is carried out after step b):
b1) recycling the organic phase comprising isobutyraldehyde separated in step b) for further reaction according to step a)

7. Process according to any of Claims 1 to 6, wherein no further purification of the distillation bottoms is carried out between step b) and c).

8. Process according to any of Claims 1 to 7, wherein step c) is conducted at a hydrogen pressure of ≥ 6 MPa to ≤ 20 MPa.

9. Process according to any of Claims 1 to 8, wherein step c) is carried out as a two-stage or multi-stage hydrogenation in a multizone reactor.

10. Process according to any of Claims 1 to 9, wherein step c) comprises a catalyst based on nickel and/or copper chromite.

## Revendications

1. Procédé de synthèse de néopentène glycol, comprenant les étapes de :
a) conversion d'isobutyraldéhyde avec du formaldéhyde en présence d'un catalyseur basique, l'isobutyraldéhyde étant présent en excès
b) purification par distillation du mélange réactionnel issu de a) de façon à ce que se forme un produit de bas de colonne contenant moins de 5 % en poids d'eau
c) hydrogénation du produit de bas de colonne en néopentène glycol.

2. Procédé selon la revendication 1, où le rapport de l'isobutyraldéhyde au formaldéhyde est ≥ 1,01:1 (en moles d'isobutyraldéhyde sur moles de formaldéhyde).

3. Procédé selon la revendication 1 ou 2, où le catalyseur basique est utilisé dans un rapport molaire ≥ 0,01 à ≤ 0,1 rapporté à l'isobutyraldéhyde.

4. Procédé selon l'une des revendications 1 à 3, dans lequel le catalyseur basique comprend de la triméthylamine et/ou une lessive alcaline.

5. Procédé selon l'une des revendications 1 à 4, dans lequel l'étape b) a lieu dans un évaporateur à couche mince.

6. Procédé selon l'une des revendications 1 à 5, comprenant en plus l'étape b1) effectuée après l'étape b) :
b1) retour de la phase organique séparée à l'étape b) contenant de l'isobutyraldéhyde pour renouveler la réaction de l'étape a).

7. Procédé selon l'une des revendications 1 à 6, dans lequel aucune nouvelle purification du produit de bas de colonne de distillation n'a lieu entre l'étape b) et l'étape c).

8. Procédé selon l'une des revendications 1 à 7, dans lequel l'étape c) a lieu sous une pression de ≥ 6 MPa à ≤ 20 MPa d'hydrogène.

9. Procédé selon l'une des revendications 1 à 8, dans lequel l'étape c) est réalisée comme hydrogénation sur au moins deux étages dans un réacteur multizone.

10. Procédé selon l'une des revendications 1 à 9, dans lequel l'étape c) comprend un catalyseur à base de nickel et/ou à base de chromite de cuivre.
